# EUROPEAN PATENT SPECIFICATION

(11) **EP 4 208 180 B1**
(45) Date of publication and mention of the grant of the patent: **05.03.2025**
(21) Application number: 21805500.2
(22) Date of filing: 03.11.2021
(51) Int. Cl.: A61K 35/741, A23L 33/105, A61K 36/82, A61P 1/00, A61P 3/06, A61P 3/08

(54) **COMPOSITION COMPRISING AKKERMANSIA MUCINIPHILA AND GREEN TEA EXTRACT**
ZUSAMMENSETZUNG ENTHALTEND AKKERMANSIA MUCINIPHILA UND GRÜNER TEE
COMPROSITION COMPRENANT AKKERMANSIA MUCINIPHILA ET DU THÉ VERT

(30) Priority: 09.11.2020 BE 202005802
(43) Date of publication of application: 12.07.2023
(73) Proprietor: The Akkermansia Company, 1435 Mont-Saint-Guinert (BE)
(72) Inventor: CANI, Patrice, 1435 Mont-Saint-Guibert (BE); BROCHET, Amandine, 1435 Mont-Saint-Guibert (BE)
(74) Representative: Willnegger, Eva
(86) International application number: PCT/EP2021/080505
(87) International publication number: WO 2022/096501

(56) References cited:
- WO-A1-2014/075745
- WO-A1-2017/042347
- DATABASE GNPD [online] MINTEL; 7 March 2019 (2019-03-07), ANONYMOUS: "Acai Lemonade High Performance Superfood Powder", XP055814097, retrieved from https://www.gnpd.com/sinatra/recordpage/6376133/ Database accession no. 6376133
- ULRIKA AXLING ET AL: "Green tea powder and Lactobacillus plantarum affect gut microbiota, lipid metabolism and inflammation in high-fat fed C57BL/6J mice", NUTRITION & METABOLISM, BIOMED CENTRAL. LONDON, GB, vol. 9, no. 1, 26 November 2012 (2012-11-26), pages 105, XP021137109, ISSN: 1743-7075, DOI: 10.1186/1743-7075-9-105
- JEONG HYUN WOO ET AL: "Green Tea Encourages Growth of Akkermansia muciniphila", vol. 23, no. 8, 1 August 2020 (2020-08-01), US, pages 841 - 851, XP055814500, ISSN: 1096-620X, Retrieved from the Internet <URL:http://dx.doi.org/10.1089/jmf.2019.4662> DOI: 10.1089/jmf.2019.4662
- DATABASE GNPD [online] MINTEL; 7 March 2019 (2019-03-07), ANONYMOUS: "Acai Lemonade High Performance Superfood Powder", XP055814097, retrieved from https://www.gnpd.com/sinatra/recordpage/6376133/ Database accession no. 6376133

## Description

### FIELD OF THE INVENTION

The present invention relates to a composition comprising *Akkermansia muciniphila* and a green tea extract. In particular, the present invention relates to a composition comprising *Akkermansia muciniphila* and a green tea extract, wherein *Akkermansia muciniphila* is pasteurized. The compositions of the present invention are useful in the prevention or treatment of a metabolic disorder selected from the group comprising obesity, metabolic syndrome, insulin-deficiency or insulin-resistance related disorders, diabetes mellitus, glucose intolerance, abnormal lipid metabolism, hyperglycemia, dyslipidemia, barrier function diseases including inflammatory bowel disease, Crohn's disease and ulcerative colitis, irritable bowel syndrome, gut contractility disorder, hypercholesterolemia, and atherogenic dyslipidemia and in particular obesity. Moreover, the compositions of the invention are useful in promoting weight loss, reducing visceral fat or improving the gut barrier function.

### BACKGROUND

Obesity and overweight are a worldwide trend, with an estimated number of obese adults of about 600 million. The prevalence of obesity-related disorders, such as, for example, diabetes, hypertension, cardiac pathologies and liver diseases. Due to these highly disabling pathologies, obesity is currently considered in Western countries as one of the most important public health problems.

Obesity is associated with metabolic dysfunctions with an impact on glucose homeostasis and lipid metabolism for example. Another comorbidity is low grade inflammatory state associated with higher blood lipopolysaccharides (LPS) levels also referred as metabolic endotoxemia.

A further comorbidity of obesity is impaired or reduced gut barrier function. Increased gut permeability leads to translocation of bacteria and/or microorganisms components into the bloodstream and organs such as the liver or the adipose tissue. Intestinal inflammation, LPS and bacterial translocation are also observed during inflammatory bowel diseases. Examples include Crohn's disease, colitis, ulcerative colitis, intestinal or colic pain and other intestinal inflammatory diseases. Interestingly, both inflammatory bowel diseases and obesity-related diseases are associated with changes in the gut microbiota composition.

The human gut is colonized by a diverse, complex and dynamic community of microbes representing over 1000 different species, which continuously interact with the host. The gut microbiota depends on host characteristics such as age, gender, genetic background. Other factors include environmental conditions such as stress, drugs, gastrointestinal surgery, infectious and toxic agents and day-to-day dietary changes.

Gut microbiota impact the development of obesity and related disorders, overweight and intestinal inflammation, or intestinal pain, dysbiosis of the microbiota can further disrupt the crosstalk between organs and the integrity of the intestinal barrier leading to symptoms.

Green tea has been described for the treatment of obesity or glycemia. For example, Kobayashi et al. (2000) (https://pubs.acs.org/doi/pdf/10.1021/jf0006832) discloses that green tea polyphenols inhibit glucose uptake. However, this document does not disclose a composition comprising *Akkermansia muciniphila* and green tea extract.

WO2013130773A2 to Kaplan discloses a method of altering relative abundance of microbiota in a subject comprising administering to the subject an effective dose of a composition consisting essentially of substantially purified Verrucomicrobia for the treatment of obesity and metabolic syndrome.

WO2014076246 to Université catholique de Louvain and Wageningen Universiteit describes the use of *Akkermansia muciniphila* for treating obesity and related disorders. However, this document does not disclose a composition comprising *Akkermansia muciniphila* and green tea extract.

EP3347030 to Université catholique de Louvain and Wageningen Universiteit describes *Akkermansia muciniphila* for use in treating a metabolic disorder in a subject in need thereof, wherein *Akkermansia muciniphila* is pasteurized. This document does not describe the combination of *Akkermansia muciniphila* with a plant extract.

Ushiroade 2019 discloses an increase of the *Akkermansia* genus in the Verrucomicrobia in high fat diet mice following the administration of the green tea compound Epigallocatechin-3-gallate. The document is available at Ushiroda et al., Green tea polyphenol (epigallocatechin-3-gallate) improves gut dysbiosis and serum bile acids dysregulation in highfat diet fed mice; J. Clin. Biochem. Nutr. | July 2019 | vol. 65 | no. 1 | 34-46, doi: 10.3164/jcbn.18116. However, this document does not disclose a composition comprising *Akkermansia muciniphila* and Green tea extract.

Sheng 2018 describes an epigallocatechin-3-gallate (EGCG) promoted increase of *Akkermansia muciniphila* effectively reduces diet-increased obesity, visceral fat, and insulin resistance. This document does not describe a composition comprising pasteurized *Akkermansia muciniphila* and green tea extract. The document is available at: Sheng et al., Obesity treatment by epigallocatechin-3-gallate-regulated bile acid signaling and its enriched Akkermansia muciniphila, FASEB J. 2018 Dec; 32(12): 6371-6384, doi: 10.1096/fj.201800370R.

US2018318323A1 to Plexus Worldwide discloses a gut microbiome modulating composition comprising green tea to stimulate the growth of Akkermansia. However, this document does not disclose a composition comprising *Akkermansia muciniphila* and green tea extract.

CN108541953 to Zhang Quingyu discloses tea polyphenols as a prebiotic to enhance the proliferation of *Akkermansia muciniphila* in cecum and ileum. This document does not disclose a composition comprising *Akkermansia muciniphila* and green tea extract.

However, there is still an urgent need to provide improved compositions for treating metabolic disorder, in particular obesity and promoting weight loss.

### SHORT DESCRIPTION OF THE INVENTION

The present inventors have surprisingly found that the association of *Akkermansia muciniphila* and a green tea extract provides superior weight loss results as compared to other plant extracts and thus is synergistic. This is particularly the case, when *Akkermansia muciniphila* is pasteurized.

Accordingly, a first aspect of the invention is a composition comprising *Akkermansia muciniphila* and a green tea extract.

In a further aspect of the invention, *Akkermansia muciniphila* is pasteurized.

A further aspect of the invention is a composition for use in the prevention or treatment of a metabolic disorder selected from the group comprising obesity, metabolic syndrome, insulin-deficiency or insulin-resistance related disorders, diabetes mellitus, glucose intolerance, abnormal lipid metabolism, hyperglycemia, dyslipidemia, barrier function diseases including inflammatory bowel disease, Crohn's disease and ulcerative colitis, irritable bowel syndrome, gut contractility disorder, hypercholesterolemia, gut barrier function disorder and atherogenic dyslipidemia.

In a further aspect of the invention, the metabolic disorder is obesity or gut barrier function disorder.

In a further aspect of the invention, the composition reduces the development of fat mass, in particular of visceral adipose tissue as compared to a non-treated subject.

In a further aspect of the invention, the composition further comprises one or more ingredients chosen from group consisting of probiotic, bacteria, yeast, microorganisms, prebiotic or a combination thereof.

In a further aspect of the invention, the composition further comprises a mineral or a vitam in or a combination thereof.

In a further aspect of the invention, the composition further comprises a pharmaceutically acceptable carrier or a food grade carrier.

In a further aspect the invention, the composition is orally administered.

In a further aspect of the invention, the pasteurized *Akkermansia muciniphila* is administered in an amount from 1.10⁴ to 1.10¹² cells per day, more preferably from 1.10⁵ cells to 1.10¹¹ cells per day, and even more preferably from 1.10⁶ to 1.10¹⁰ cells per day, preferably as expressed in TFU by flow cytometry.

In a further aspect of the invention, the green tea extract is administered in an amount of 30 mg to 600 mg per day. In another embodiment, the green tea extract is administered in an amount up to 1000 mg or even 2000 mg per day.

In a further aspect of the invention, the green tea has 10 or even less 5% w% or more, preferably 50 w% or more of Epigallocatechin gallate (EGCG).

In a further aspect of the invention, the green tea extract is administered in an amount that delivers 100 mg to 600 mg per day EGCG. In a further aspect of the invention, the green tea extract is administered in an amount that delivers 200 mg to 300 mg per day of EGCG.

In a further aspect of the invention, the composition is a cosmetic composition, a nutritional composition, a food product, a dietary complement, a medical food or a medicament.

In a further aspect of the invention, the composition further comprises a second plant extract.

In a further aspect of the invention, the second plant extract is chosen from the group consisting of *Aronia melanocarpa, Emblica officinalis, Olea Europa, Citrus bergamia, Vaccinium macrocarpon, Myrciaria dubia,* red Panax ginseng, *Vaccinium oxycoccos, Vaccinium macrocarpon.*

A further aspect of the invention is a method of production of the composition of the invention comprising the steps of
a. Culturing cells of the genus Akkermansia;
b. Pasteurizing the cultured cells obtained of step a);
c. Adding a cryoprotectant and a pH buffer;
d. Freeze-drying the pasteurized cells of step c);
e. Mixing the freeze-dried powder with a green tea extract; and
f. Optionally producing capsules, gel capsules or soft gel capsules from the mix of step e).

A further aspect of the invention is a composition according to any of the preceding claims for promoting weight loss or the gut barrier function in a subject in need thereof.

A further aspect of the invention is a method of treating or preventing a metabolic disorder in a subject in need thereof, wherein the method comprises administering to the subject an effective amount in particular without treatment-limiting side-effects, of a composition comprising *Akkermansia muciniphila* and green tea extract. In a preferred embodiment, *Akkermansia muciniphila* is pasteurized.

### DEFINITIONS

"Treatment" means reducing or alleviating at least one adverse effect or symptom of a disease, disorder or condition. This term thus refers to both therapeutic treatment and prophylactic or preventative measures.

"Prevention" means preventing in the sense of keeping from happening or reducing the risk of a disease or condition.

"Effective amount" or "therapeutically effective amount" refers to level or amount of agent that is aimed at, without causing significant negative or adverse side effects to the target, delaying or preventing the onset of a metabolic disorder, slowing down or stopping the progression, aggravation, or deterioration of one or more symptoms of the metabolic disorder; bringing about ameliorations of the symptoms of the metabolic disorder; reducing the severity or incidence of the metabolic disorder; curing the metabolic disorder; or restoring the normal amount and/or proportion of *Akkermansia muciniphila* in the gut of the subject to be treated.

*"Akkermansia muciniphila"* refers to the mucin-degrading bacteria identified by Derrien (Derrien et al., 2004. Int. J. Syst. Evol. Microbiol. 54:1469-1476). Cells are oval-shaped, non-motile and stain Gram-negative. *Akkermansia muciniphila* may also be referred as Akkermansia spp. or Akkermansia-like bacteria. It belongs to the Verrucomicrobia phylum. It is generally accepted that strains with a nucleotide similarity as experimentally determined by DNA-DNA hybridization of about 70% can be considered as the same species - this corresponds to an average nucleotide identity (ANI) of approximately 95%.

In one embodiment, *Akkermansia muciniphila* also comprises fragments of *Akkermansia muciniphila.*

"Pasteurized *Akkermansia muciniphila"* refers to *Akkermansia muciniphila* submitted to a heating treatment. In one embodiment, pasteurized *Akkermansia muciniphila* refers to *Akkermansia muciniphila* which was heated at a temperature from 50°C to 100°C for at least 10 minutes.

"TFU" or "Total Fluorescent Units" means the number of cells as determined by their fluorescence radiance after staining. In a preferred embodiment, the TFU is measured flow cytometry. For example, in a first step, aliquots of biomass batches are rehydrated in PBS and stained with Syto 9 and propidium iodide according manufacturer protocol (LIVE/DEAD^{®} BacLight TM Bacterial Viability Kit, Thermofisher). The TFU may then be obtained by analyzing the stained samples on Attune NxT flow cytometer. In general, TFU are expressed as total fluorescent unit per gram or per ml, preferably per gram.

"Green tea extract" means any extract of plant leaves or buds of *Camellia sinensis.* Green tea extract is in general rich in polyphenols, in particular epigallocatechin gallate (EGCG), epicatechin gallate, epicatechins and flavanols. Green tea extract may be obtained by alcoholic extraction or water extraction, concentrated or not and processed into a liquid or powder.

"Probiotics" refers to live microorganisms which, when administered in an effective amount, provide a beneficial effect on the health or well-being of a subject. In one embodiment, these health benefits are associated with improving the balance of human or animal microbiota in the gastro-intestinal tract, or restoring normal microbiota.

"Prebiotic" refers to a substance, such as, for example, a substance which may not be digested by humans, but which modulates composition and/or activity of the gut microbiota through its metabolization by microorganisms in the gut, thus conferring a beneficial physiological effect on the host.

"Subject" refers to an animal, preferably a mammal, more preferably a human or an animal.

"Overweight" refers to a subject situation wherein said subject has a Body Mass Index (BMI) of 25 or more. As used herein, BMI is defined as the individual's body mass (in kg) divided by the square of his/her height (in meter).

"Obesity" refers to a subject situation wherein said subject has a BMI superior or equal to 30.

"Fragment" may refer to cellular components, metabolites, secreted molecules, vesicles and compounds resulting from the metabolism of *Akkermansia muciniphila* and the like. Fragments may be obtained, for example, by recovering the supernatant of a culture of *Akkermansia muciniphila* or by extracting cell components or cell fractions, metabolites or secreted compounds from a culture of *Akkermansia muciniphila.*

### DETAILED DESCRIPTION OF THE INVENTION

The applicant herein shows that the beneficial effects on metabolism observed after administration of a composition comprising *Akkermansia muciniphila* and a green tea extract, as showed in the examples for *Akkermansia muciniphila.*

Therefore, this invention relates to a composition comprising *Akkermansia muciniphila* and a green tea extract for treating, or for use in treating, metabolic disorders in a subject in need thereof.

As used herein, a metabolic disorder is a disorder related to an altered metabolic homeostasis, such as, for example, an altered glucose or lipid homeostasis.

In one embodiment of the invention, said metabolic disorder is obesity.

Examples of other metabolic disorders include, but are not limited to, metabolic syndrome, insulin-deficiency or insulin-resistance related disorders, Diabetes Mellitus including Type 1 Diabetes or Type 2 Diabetes, glucose intolerance, abnormal lipid metabolism, atherosclerosis, hypertension, pre-eclampsia, cardiac pathology, stroke, non-alcoholic fatty liver disease, hyperglycemia, hepatic steatosis, dyslipidemia, dysfunction of the immune system associated with overweight and obesity, liver diseases such as, for example, fibrosis associated with obesity, or abnormal liver functions, including changes in bile production, inflammatory, immune and barrier function diseases such as, for example, inflammatory bowel disease, including Crohn's disease and ulcerative colitis, and irritable bowel syndrome, cardiovascular diseases, high cholesterol, elevated triglycerides, asthma, sleep apnea, osteoarthritis, neuro-degeneration, gallbladder disease, syndrome X, inflammatory and immune disorders, atherogenic dyslipidemia and cancer.

In one embodiment, hypercholesterolemia corresponds to a plasma cholesterol concentration superior or equal to 2 g/L or 5 mmol/L. In another embodiment, hypercholesterolemia corresponds to a ratio plasma concentration of total cholesterol: plasma concentration of HDL (high density lipoprotein cholesterol) superior or equal to 4.5:1, preferably 5:1.

In to one embodiment, pasteurized *Akkermansia muciniphila* of the invention are non-viable cells. As used herein, "non-viable cells" means cells that are not able to proliferate. Examples to visualize or counts cells of *Akkermansia muciniphila* have been provided by Derrien et al. (2008. Appl. Environ. Microbiol. 74:1646-8), Derrien et al. (2011. Frontiers Microbiol. 2:166-175) or Reunanen et al. (2015. Appl. Environ. Microbiol. 81(11):3655-62).

The composition of the invention may also comprise a pharmaceutically acceptable excipient or a food grade carrier, for example solvents, dispersion media, coatings, isotonic and absorption delaying agents and the like. For human administration, preparations should meet general safety and purity standards as required by FDA Office of Biologics standards.

The present invention also relates to a medicament, medical food, food or dietary compliment comprising an effective amount of *Akkermansia muciniphila* or a fragment thereof and green tea extract.

The present invention also relates to a method for treating or preventing a metabolic disorder in a subject in need thereof, wherein said method comprises administering an effective amount of *Akkermansia muciniphila* or a fragment thereof and green tea extract to the subject in need thereof.

Another object of the invention is a method for restoring a normal proportion of *Akkermansia muciniphila,* fragments or other active compounds of *Akkermansia muciniphila* in the gut of a subject in need thereof, wherein said method comprises administering an effective amount of *Akkermansia muciniphila* or a fragment thereof and green tea extract to the subject.

In one embodiment of the invention, the composition of the invention is administered at least once a week, preferably at least twice a week, more preferably at least three times a week, and even more preferably at least four times a week. In another embodiment, the composition of the invention is administered at least once a day, and preferably at least twice a day.

In one embodiment, the composition of the invention is administered during 1 week, preferably during 2, 3, 4, 5, 6, 7 or 8 weeks or more or even permanently.

In one embodiment, the composition of the invention is administered for a period that lasts until the desired outcome is achieved, for example weight loss, the metabolic disorder treatment, or the decrease of cholesterol plasma level.

In one embodiment of the invention, the daily dosage of *Akkermansia muciniphila* administered is from 1.10² to about 1.10¹⁵ TFU/day, preferably from about 1.10⁴ to about 1.10¹²TFU/day, more preferably from about 1.10⁵ to about 1.10¹¹ TFU/day and even more preferably from about 1.10⁶ to about 1.10¹⁰ TFU/day.

In another embodiment of the invention, the daily dosage of pasteurized *Akkermansia muciniphila* is from 1.10⁶ to about 1.10¹² cells/day, preferably from about 1.10⁸ to about 1.10¹⁰ cells/day, more preferably from about 1.10⁹ to about 1.10¹⁰ cells/day.

The present invention also relates to the cosmetic use of pasteurized *Akkermansia muciniphila* or a fragment thereof for promoting weight loss in a subject.

Another object of the invention is thus a cosmetic composition comprising a cosmetically effective amount of pasteurized *Akkermansia muciniphila* or a fragment thereof and green tea extract, and the use thereof for promoting weight loss in a subject. As used herein, a "cosmetically effective amount" refers to the amount of a cosmetic composition necessary and sufficient for promoting a cosmetic effect, such as, for example, for inducing weight loss in a subject.

In one embodiment of the invention, the composition, the pharmaceutical composition, the cosmetic composition or the medicament further comprises additional probiotic strains or species, such as, for example, bacterial probiotic strains or species. In another embodiment, the further strains are pasteurized. These probiotics include bacteria, or fungal strains or species, preferably yeast strains or species. In one embodiment, said additional probiotic strains or species are selected from those naturally present in the gut of the subject, preferably in the human gut, more preferably in the gut of healthy human subjects.

Examples of bacterial probiotic strains or species that may be used in the present invention include, but are not limited to *Lactobacillus, Lactococcus, Bifidobacterium, Veillonella, Desemzia, Christensenella, Allobaculum, Coprococcus, Collinsella, Citrobacter, Turicibacter, Sutterella, Subdoligranulum, Streptococcus, Sporobacter, Sporacetigenium, Ruminococcus, Roseburia, Proteus, Propionobacterium, Leuconostoc, Weissella, Pediococcus, Streptococcus, Prevotella, Parabacteroides, Papillibacter, Oscillospira, Melissococcus, Dorea, Dialister, Clostridium, Cedecea, Catenibacterium, Butyrivibrio, Buttiauxella, Bulleidia, Bilophila, Bacteroides, Anaerovorax, Anaerostipes, Anaerofilum, Enterobacteriaceae, Fermicutes, Atopobium, Alistipes, Acinetobacter, Slackie, Shigella, Shewanella, Serratia, Mahella, Lachnospira, Klebsiella, Idiomatina, Fusobacterium, Faecalibacterium, Eubacterium, Enterococcus, Enterobacter, Eggerthella, Dysosmobacter, or Anaerobacterium.*

Preferred probiotic strains are *Lactobacillus, Lactococcus, Bifidobacterium, Christensenella, Clostridium, Anaerostipes, Faecalibacterium, Eubacterium, Enterococcus, Enterobacter, Eggerthella, Dysosmobacter,* or *Anaerobacterium.*

Examples of prokaryote strains or species that may be used in the present invention include, but are not limited to Archaea, Firmicutes, Verrucomicrobia, *Christensenella,* Bacteroidetes (such as, for example, *Allistipes, Bacteroides ovatus, Bacteroides splachnicus, Bacteroides stercons, Parabacteroides, Prevotella ruminicola, Porphyromondaceae,* and related genus), Proteobacteria, Betaproteobacteria (such as, for example, *Aquabacterium* and Burkholderia), Gammaproteobacteria (such as, for example, *Xanthomonadaceae*), Actinobacteria (such as, for example, *Actinomycetaceae* and *Atopobium*), Fusobacteria, Methanobacteria, Spirochaetes, Fibrobacteres, Deferribacteres, *Deinococcus, Thermus,* Cyanobacteria, Methanobrevibacteria, *Peptostreptococcus, Ruminococcus, Coprococcus, Subdolingranulum, Dorea, Bulleidia, Anaerofustis, Gemella, Roseburia, Dialister, Anaerotruncus, Staphylococcus, Micrococcus,* Propionobacteria, Enterobacteriaceae, *Faecalibacterium, Bacteroides, Parabacteroides, Prevotella, Eubacterium,* Bacilli (such as, for example, *Lactobacillus salivarius* and related species, *Aerococcus, Granulicatella, Streptococcus bovis* and related genus and *Streptococcus intermedius* and related genus), *Clostridium* (such as, for example, *Anaerobutyricum hallii, Eubacterium limosum, Anaerobutyricum soehngenii* and related genus) and *Butyrivibrio.*

Examples of fungal probiotic strains or species, preferably yeast probiotic strains or species that may be used in the present invention include, but are not limited *Ascomycetes, Zygomycetes* and *Deuteromycetes,* preferably from the groups *Aspergillus, Torulopsis, Zygosaccharomyces, Hansenula, Candida, Saccharomyces, Clavispora, Bretanomyces, Pichia, Amylomyces, Zygosaccharomyces, Endomycess, Hyphopichia, Zygosaccharomyces, Kluyveromyces, Mucor, Rhizopus, Yarrowia, Endomyces, Debaryomyces,* and/or *Penicillium.*

In one embodiment of the invention, the only one microbial strain or species, preferably bacterial strain or species, comprised in the composition, pharmaceutical composition, cosmetic composition or medicament is *Akkermansia muciniphila.*

In one embodiment of the invention, the composition, pharmaceutical composition, cosmetic composition or medicament consists of pasteurized *Akkermansia muciniphila.*

In one embodiment of the invention, the composition, the pharmaceutical composition, the cosmetic composition or the medicament further comprises a prebiotic.

Examples of prebiotics that may be used in the present invention include, but are not limited to, inulin and inulin-type fructans, oligofructose, beta-glucans, xylose, arabinose, arabinoxylan, ribose, galactose, rhamnose, cellobiose, fructose, lactose, salicin, sucrose, glucose, esculin, tween 80, trehalose, maltose, mannose, mellibiose, mucus or mucins, raffinose, fructooligosaccharides, galacto-oligosaccharides, amino acids, alcohols, fermentable carbohydrates and any combinations thereof.

Other non-limiting examples of prebiotics include water-soluble cellulose derivatives, water-insoluble cellulose derivatives, unprocessed oatmeal, metamucil, bran, and any combinations thereof.

Examples of water-soluble cellulose derivatives include, but are not limited to, methylcellulose, methyl ethyl cellulose, hydroxyethyl cellulose, ethyl hydroxyethyl cellulose, cationic hydroxyethyl cellulose, hydroxypropyl cellulose, hydroxyethyl methylcellulose, hydroxypropyl methylcellulose, and carboxymethyl cellulose.

The composition of the invention may be administered by oral administration, rectal administration, administration via esophagogastroduodenoscopy, administration via colonoscopy, administration using a nasogastric or orogastric tube.

The composition may be administered orally as tablets, pills, capsules, soft gelatin capsules, sugarcoated pills, or dispersing tablets, effervescent tablets or other solids.

The composition may be administered orally as a liquid, a drinkable solution or a liposome.

In one embodiment, the composition of the invention further comprises excipients, diluent and/or carriers selected with regard to the intended route of administration. Examples of excipients, diluent and/or carriers include, but are not limited to, water, phosphate buffer saline, anaerobic phosphate buffer saline, sodium bicarbonate, juice, milk, yogurt, infant formula, dairy product, coloring agents, such as, for example, titane dioxide (E171), iron dioxide (E172) and brilliant black BN (E151); flavoring agents; thickeners, such as, for example, glycerol monostearate; sweeteners; coating agents, such as, for example, refined colza oil, soya oil, peanut oil, soya lecithin or fish gelatin; diluting agents, such as, for example, lactose, monohydrated lactose or starch; binding agents, such as, for example, povidone, pregelatinized starch, gums, saccharose, polyethylene glycol (PEG) 4000 or PEG 6000; disintegrating agents, such as, for example, microcrystalline cellulose or sodium carboxymethyl starch, such as, for example, sodium carboxymethyl starch type A; lubricant agents, such as, for example, magnesium stearate; flow agent, such as, for example, colloidal anhydrous silica, etc.

In one embodiment of the invention, the composition of the invention is a pharmaceutical composition.

In another embodiment, the composition of the invention is a food additive, drink additive, dietary supplement, nutritional product, medical food or nutraceutical composition.

Obesity and related disorders are associated with an increased gut permeability and with impaired mucus production, epithelium barrier, immune system and/or antibacterial compounds production by the subject.

Accordingly, the composition of the present invention may be used for decreasing gut permeability, for restoring impaired mucus production, for restoring epithelium barrier, for restoring immune system, or for restoring the production of antibacterial compounds or a combination thereof.

Moreover, the composition of the invention may be useful in controlling gut barrier function. Accordingly, another aspect is a method for controlling gut barrier function comprising administering an effective or cosmetically effective amount of the composition of the invention to a subject in need thereof.

In one embodiment, the composition of the invention regulates mucus layer thickness which may be decreased in obesity or other metabolic disorders. In another embodiment, the administration of the composition of the invention induces the production of colon antimicrobial peptides, such as, for example, RegIIIgamma. In another embodiment, the administration of composition of the invention induces the production of compounds of the endocannabinoids family, such as, for example, acylglycerols selected from the group comprising 2-oleoylglycerol, 2-palmitoylglycerol and 2-arachidonoylglycerol. In another embodiment, the administration of the composition of the invention regulates mucus turnover.

In one embodiment, the administration of the composition of the invention to a subject increases satiety in said subject. Consequently, according to this embodiment, the method of the invention increases satiety or decreases food intake in a subject, thereby inducing durable weight loss in the subject, and thereby treating metabolic disorders in said subject, such as, for example, obesity related metabolic disorders.

### SHORT DESCRIPTION OF THE DRAWINGS

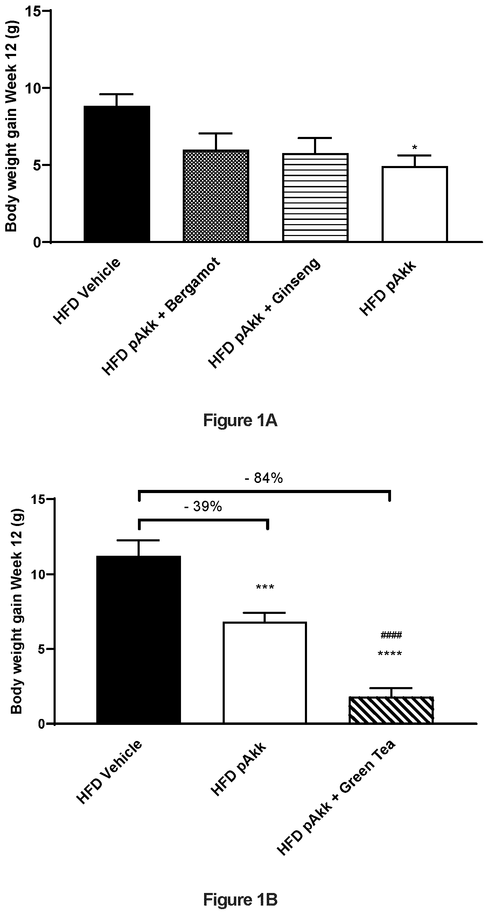

Figures 1 shows the body weight at week 12 of the oral administration of vehicle or pasteurized *A. muciniphila* supplemented or not with plant extracts (Figure 1A and 1B) on body weight gain at week 12 in a DIO model (45 kcal% lipids, 12 weeks). ***p < 0.001, ****p < 0.0001 vs HFD Vehicle; ^{###}p < 0.001, ^{####}p < 0.001 vs HFD pAkk (p values obtained using 1-way ANOVA followed by Bonferroni's post-hoc test).

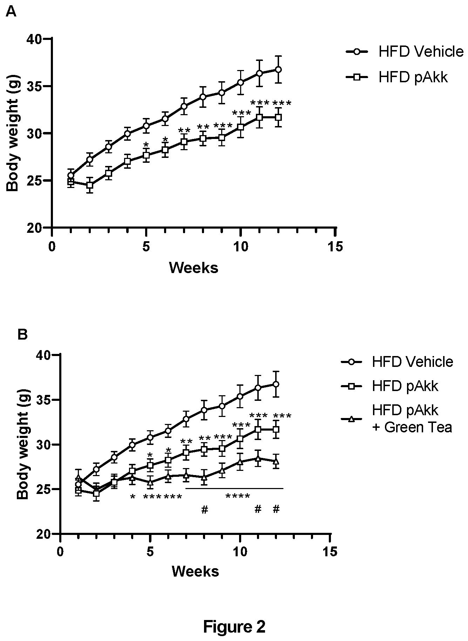

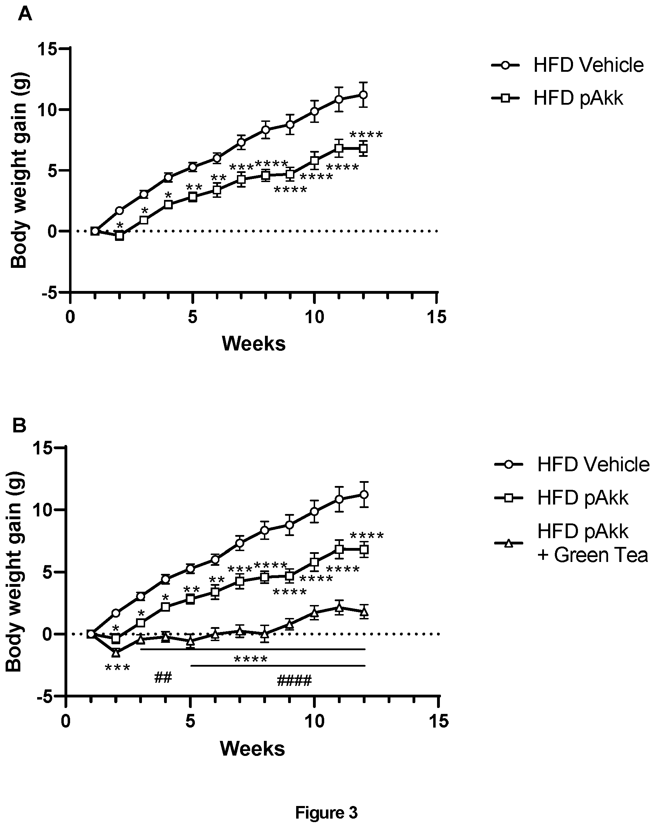

Figures 2A, 2B, 3A and 3B show the effect of pasteurized *Akkermansia muciniphila* alone or in combination with plant extracts on body weight and body weight gain in a DIO model (45 kcal% lipids, 12 weeks).
Figure 2 shows the body weight over time during oral administration of vehicle (circles) or pasteurized *A. muciniphila* alone (squares) or pasteurized *A. muciniphila* with Green tea (triangles) on body weight gain over time in a DIO model (45 kcal% lipids, 12 weeks). *p < 0.05, **p < 0.01, ***p < 0.001, ****p < 0.0001 vs HFD Vehicle; ^{#}p < 0.05, ^{##}p < 0.01, ^{####}p < 0.0001vs HFD pAkk. (p values obtained using 1 -way ANOVA followed by Bonferroni's post-hoc test).
Figure 3 shows body weight gain over time during oral administration of vehicle (circles) or pasteurized *A. muciniphila* alone (squares) or pasteurized *A. muciniphila* with Green tea (triangles) on body weight gain over time in a DIO model (45 kcal% lipids, 12 weeks). *p < 0.05, **p < 0.01, ***p < 0.001, ****p < 0.0001 vs HFD Vehicle; ^{#}p < 0.05, ^{##}p < 0.01, ^{####}p < 0.0001vs HFD pAkk. (p values obtained using 1 -way ANOVA followed by Bonferroni's post-hoc test).

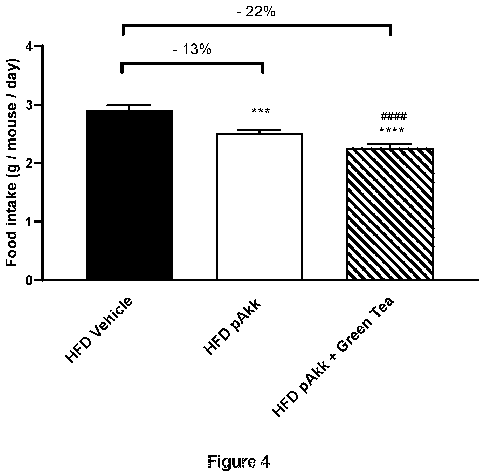

Figure 4 shows food intake during oral administration of vehicle or pasteurized *A. muciniphila* supplemented or not with Green tea on food intake. ***p < 0.001, ****p < 0.0001 vs HFD Vehicle; ^{####}p < 0.0001vs HFD pAkk (p values obtained using 1-way ANOVA followed by Bonferroni's post-hoc test).
Figures 4A, 4B, 4C, 4D, and 4E show the effect of a composition comprising pasteurized *Akkermansia muciniphila* and green tea extract on the gut barrier function in a DIO model (45 kcal% lipids, 12 weeks).

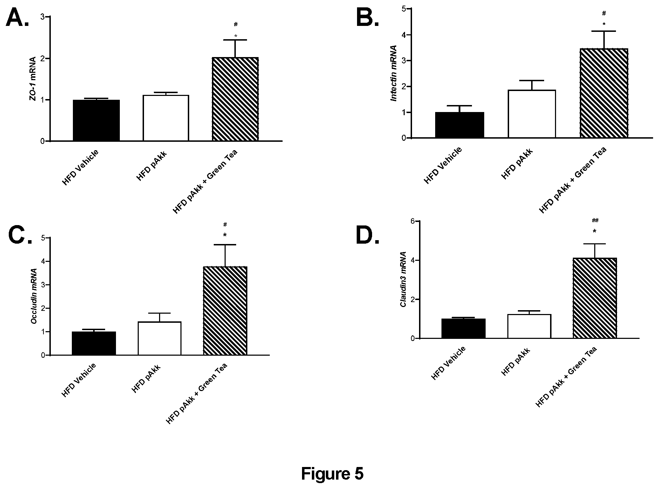

Figure 5 shows the gene expression of intestinal barrier integrity biomarkers in the colon during oral administration of vehicle or pasteurized *A. muciniphila* supplemented or not with Green Tea at week 12 on various markers of the intestinal barrier integrity in the colon (PCR analysis of fold change vs. HFD Vehicle group) (A) ZO-1 expression, (B) Intectin expression, (C) Occludin expression, (D) Claudin3 expression. *p < 0.05 vs HFD Vehicle; ^{#}p < 0.05, vs HFD pAkk. (p values obtained using 1 -way ANOVA followed by Bonferroni's post-hoc test).

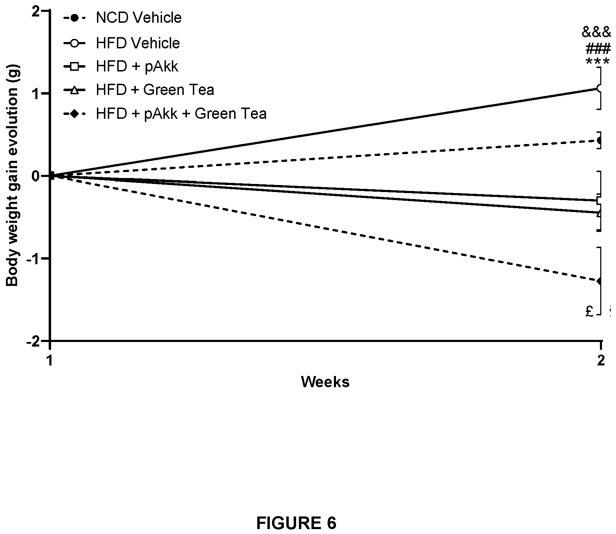

Figure 6: Body weight gain after 1 week of treatment. Effects of an oral administration of vehicle or pasteurized Akkermansia muciniphila (pAkk) supplemented or not with Green Tea extract on body weight after 1 week of treatment. &&&& p < 0.001 HFD vs HFD pAkk, **** p < 0.0001 HFD vs HFD + Green Tea, ####p < 0.0001 HFD vs pAkk + Green Tea, £ p < 0.05 pAkk vs pAkk + Green Tea, § p < 0.05 Green tea vs pAkk + Green Tea. The associated p-values obtained using 2-way ANOVA followed by Bonferroni's post-hoc test. The other significant comparisons are not presented. The associated p-values obtained using 2-way ANOVA followed by Bonferroni's post-hoc test. The other significant comparisons are not presented.

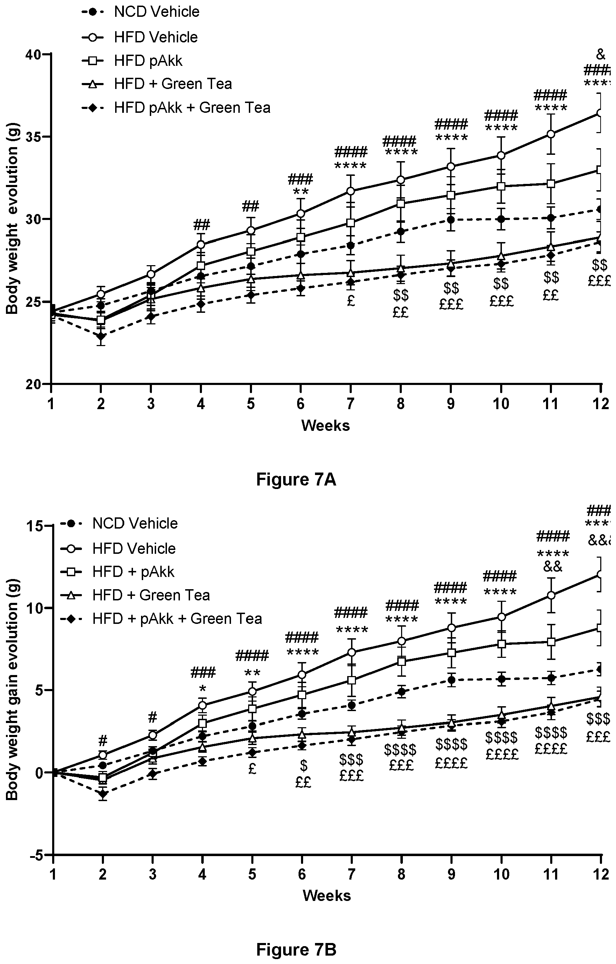

Figure 7: Body weight monitoring during the 12 weeks of treatment. Effects of an oral administration of vehicle or pasteurized Akkermansia muciniphila (pAkk) supplemented or not with Green Tea extract on (A) Body weight evolution over time, (B) Body weight gain evolution over time. & p < 0.05, && p < 0.01, &&& p < 0.001 HFD vehicle vs HFD pAkk, * p < 0.05, **p < 0.01, ****p < 0.0001 HFD vs HFD + Green Tea, # p < 0.05, ## p < 0.01, ### p < 0.001, #### p < 0.0001 HFD vs HFD pAkk + Green Tea, £ p < 0.05, ££ p < 0.01, £££ p < 0.001, ££££ p < 0.0001 HFD pAkk vs HFD pAkk + Green Tea, $ p < 0.05, $$ p < 0.01 HFD, $$$ p < 0.001, $$$$ p < 0.0001 pAkk vs HFD Green Tea. The associated p-values obtained using 2-way ANOVA followed by Bonferroni's post-hoc test. The other significant comparisons are not presented.

### DETAILED DESCRIPTION OF THE DRAWING

Figures 1, 2, 3, 4, and 5 show the in vivo effect of pasteurized *Akkermansia muciniphila* (in the Figures referred to as "pAkk") alone or in combination with plant extracts on body weight, body weight gain, food intake in mice and the gut barrier function in High Fat Diet-fed mice, hereinafter referred to as "HFD".

Nine-week-old male C57BL/6J mice (Charles River Laboratory, l'Arbresle, France) were allowed to at least 5 days of acclimatization period after the arrival. The animals were housed in ventilated and enriched cages (48 x 37.5 x 21 cm ³) throughout the experimental phase. Animals' cages litters were changed once weekly. Mice were housed in groups of 5 animals on a normal light cycle (at 07:00 pm lights off), 22 ± 2 °C and 50 ± 10% relative humidity. Housing parameters were daily recorded. During the acclimation phase, standard diet (RM1 (E) 801492, SDS) and tap water were provided ad libitum. During the experimental phase (12 weeks), High fat diet 45 kcal% (Research Diet #12451) (HFD group) and tap water were provided ad libitum. Mice were treated daily with an oral gavage of 180 µL of vehicle, or 180 µL *A. muciniphila* solution alone or 180 µL of *A. muciniphila* solution ± each plant extract (Bergamote: 125 mg/kg; Ginseng: 50 mg/kg or Green tea: 300 mg/kg) for the 12 weeks of HFD treatment.

The body weight, the food and water intake were assessed weekly.

Proximal colon was homogenized individually and total RNA from tissues were prepared using TRIReagent (Sigma Aldrich) and RNeasy 96 QIAcube HT kit (Qiagen). Quantification analysis of total RNA was performed by analyzing 1 µl of each sample in Nanodrop (Ozyme). cDNA was prepared by reverse transcription of 1 µg total RNA using a RT iScript kit (Biorad). Real-time PCR was performed with the Via7 real-time PCR system and software (Applied biotechnology) using SYBR Green Real-Time PCR Master Mixes (Biorad) for detection. RPL19 was chosen as the housekeeping gene. All samples were performed in duplicate, and data were analyzed according to the 2ΔΔCT method. The identity and purity of the amplified product were assessed by melting curve analysis at the end of amplification.

Surprising, of the plant extracts green tea was the one, that combined with pasteurized *Akkermansia muciniphila* showed superior results on weight, weight gain (figure 1, 2 and 3), and the gut barrier function (Figure 5). Indeed, body weight gain was significantly lower for the combination of pasteurized *Akkermansia muciniphila* and green tea extract than with pasteurized *Akkermansia muciniphila* alone. These results indicate a potentiator or synergistic effect of green tea extract when combined with pasteurized *Akkermansia muciniphila.*

The association of pasteurized *Akkermansia muciniphila* and green tea extract also improved the barrier function with a more pronounced effect on ZO-1, intectin, occluding and Claudin-3 (see Figure 5).

Figures 6 and 7A and 7B show the in vivo effect of pasteurized Akkermansia muciniphila (in the Figures referred to as "pAkk") alone or in combination with Green Tea on body weight or body weight gain High Fat Diet-fed mice, hereinafter referred to as "HFD".

Nine-week-old male C57BL/6J mice (Charles River Laboratory, l'Arbresle, France) were allowed to at least 5 days of acclimatization period after the arrival. The animals were housed in ventilated and enriched cages (48 x 37.5 x 21 cm³) throughout the experimental phase. Animals' cages litters were changed once weekly. Mice were housed in groups of 5 animals on a normal light cycle (at 07:00 pm lights off), 22 ± 2 °C and 50 ± 10% relative humidity. Housing parameters were daily recorded. During the acclimation phase, standard diet (RM1 (E) 801492, SDS) and tap water were provided ad libitum. During the experimental phase (12 weeks), High fat diet 45 kcal% (Research Diet #12451) (HFD groups) and tap water were provided ad libitum, control mice receiving normal chow diet (RM1 (E) 801492, SDS) (NCD group). Mice were treated daily with an oral gavage of 180 µL of vehicle, or 180 µL pasteurized A. muciniphila solution (2.108 TFU/day) alone or in combination with Green Tea extract (300 mg/kg) for the 12 weeks of HFD treatment.

At the beginning of the protocol, 13 mice per group have been ordered to compensate for a potential mortality linked to the force-feeding or fight. All the mice were used to do the statistical analyses except for the pAkk group and pAkk + Green Tea group where respectively the 3-09 and 5-03 mice died due to the force-feeding.

After one week of treatment, HFD + pAkk, HFD + Green Tea extract, and the HFD + pAkk + Green Tea showed a body weight gain lower than that of the HFD Vehicle group (Fig. 1). Furthermore, the combination of pAkk with Green Tea showed increased efficacy with a significantly lowered body weight gain compared to single-agent treatment (1,24 g reduction in the combination vs. 0,36 and 0,44g reduction in the pAkk and GT group respectively, p < 0.05) (Fig. 1). This effect is indeed synergistic as the combination of pAkk and Green Tea achieved more effect than the sum of their parts' (Wooten et al., 2021).

Over time, the association of pAkk + Green Tea is still more effective than pAkk or Green Tea alone on body weight or bodyweight gain with a significant effect starting week 2 until the end of the protocol for body weight gain (Fig. 2).

## Claims

1. Composition comprising *Akkermansia muciniphila* and a green tea extract.

2. Composition according to claim 1, wherein *Akkermansia muciniphila* is pasteurized.

3. Composition according any of the preceding claims for use in the prevention or treatment of gut barrier dysfunction or of a metabolic disorder selected from the group comprising obesity, metabolic syndrome, insulin-deficiency or insulin-resistance related disorders, diabetes mellitus, glucose intolerance, abnormal lipid metabolism, hyperglycemia, dyslipidemia, barrier function diseases including inflammatory bowel disease, Crohn's disease and ulcerative colitis, irritable bowel syndrome, gut contractility disorder, hypercholesterolemia, and atherogenic dyslipidemia.

4. Composition for use according to claim 3, wherein the metabolic disorder is obesity.

5. Composition according to claims 1 or 2 or composition for use according to claims 3 or 4, further comprising one or more ingredients chosen from group consisting of probiotic, bacteria, yeast, microorganisms, prebiotic or a combination thereof.

6. Composition according to claims 1 or 2 or composition for use according to claims 3 to 5, wherein the composition further comprising a mineral or a vitamin or a combination thereof.

7. Composition according to claims 1 or 2 or composition for use according to claims 3 to 6, wherein the composition further comprises a pharmaceutically acceptable carrier or a food grade carrier.

8. Composition according to claims 1 or 2 or composition for use according to claims 3 to 7, wherein the composition is orally administered.

9. Composition according to claims 1 or 2 or composition for use according to claims 3 to 8, wherein the pasteurized *Akkermansia muciniphila* is administered in an amount from 1.10⁴ to 1.10¹² cells per day, more preferably from 1.10⁵ cells to 1.10¹¹ cells per day, and even more preferably from 1.10⁶ to 1.10¹⁰ cells per day.

10. Composition according to claims 1 or 2 or composition for use according to claims 3 to 9, wherein the green tea extract is administered in an amount of 30 mg to 300 mg per day, preferably from 100 mg to 200 mg per day of Epigallocatechin gallate (EGCG).

11. Composition according to claims 1 or 2 or composition for use according to claims 3 to 10, wherein the composition is a cosmetic composition, a nutritional composition, a food product, a dietary complement, a medical food or a medicament.

12. Composition according to claims 1 or 2 or composition for use according to claims 3 to 11, wherein the composition further comprises a second plant extract.

13. Composition according to claims 1 or 2 or composition for use according to claims 3 to 12, wherein the second plant extract is chosen from the group consisting of *Aronia melanocarpa, Emblica officinalis, Olea Europa, Citrus bergamia, Vaccinium macrocarpon, Myrciana dubia,* red Panax ginseng, *Vaccinium oxycoccos, Vaccinium macrocarpon.*

14. Method of production of composition according to claims 1 or 2 comprising the steps of
a. Culturing cells of the genus *Akkermansia;*
b. Pasteurizing the cultured cells obtained of step a);
c. Adding a cryoprotectant and a pH buffer;
d. Freeze-drying the pasteurized cells of step c);
e. Mixing the freeze-dried powder with a green tea extract; and
f. Optionally producing capsules or gel capsules from the mix of step e).

15. Use of the composition according to claims 1 or to 2 for promoting weight loss or for reducing visceral fat.

## Patentansprüche

1. Zusammensetzung, die *Akkermansia muciniphila* und einen Grüntee-Extrakt enthält.

2. Zusammensetzung nach Anspruch 1, wobei *Akkermansia muciniphila* pasteurisiert ist.

3. Zusammensetzung nach einem der vorhergehenden Ansprüche zur Verwendung bei der Vorbeugung oder Behandlung von Darmbarrieredysfunktion oder einer Stoffwechselstörung, ausgewählt aus der Gruppe, die Fettleibigkeit, metabolisches Syndrom, mit Insulinmangel oder Insulinresistenz verbundene Störungen, Diabetes mellitus, Glukoseintoleranz, anormalem Lipidstoffwechsel, Hyperglykämie, Dyslipidämie, Barrierefunktionserkrankungen einschließlich entzündlicher Darmerkrankungen, Morbus Crohn und Colitis ulcerosa, Reizdarmsyndrom, Darmkontraktilitätsstörung, Hypercholesterinämie und atherogener Dyslipidämie.

4. Zusammensetzung zur Verwendung nach Anspruch 3, wobei die Stoffwechselstörung Adipositas ist.

5. Zusammensetzung nach Anspruch 1 oder 2 oder Zusammensetzung zur Verwendung nach Anspruch 3 oder 4, ferner umfassend einen oder mehrere Bestandteile, ausgewählt aus der Gruppe bestehend aus Probiotika, Bakterien, Hefe, Mikroorganismen, Präbiotika oder einer Kombination davon.

6. Zusammensetzung nach einem der Ansprüche 1 oder 2 oder Zusammensetzung zur Verwendung nach einem der Ansprüche 3 bis 5, wobei die Zusammensetzung außerdem ein Mineral oder ein Vitamin oder eine Kombination davon enthält.

7. Zusammensetzung nach Anspruch 1 oder 2 oder Zusammensetzung zur Verwendung nach Anspruch 3 bis 6, wobei die Zusammensetzung ferner einen pharmazeutisch akzeptablen Träger oder einen Träger mit Lebensmittelqualität umfasst.

8. Zusammensetzung nach einem der Ansprüche 1 oder 2 oder Zusammensetzung zur Verwendung nach einem der Ansprüche 3 bis 7, wobei die Zusammensetzung oral verabreicht wird.

9. Zusammensetzung nach Anspruch 1 oder 2 oder Zusammensetzung zur Verwendung nach Anspruch 3 bis 8, wobei die pasteurisierte *Akkermansia muciniphila* in einer Menge von 1.10⁴ bis 1.10¹² Zellen pro Tag, weiter bevorzugt von 1.10⁵ Zellen bis 1.10¹¹ Zellen pro Tag und noch weiter bevorzugt von 1.10⁶ bis 1.10¹⁰ Zellen pro Tag verabreicht wird.

10. Zusammensetzung nach Anspruch 1 oder 2 oder Zusammensetzung zur Verwendung nach Anspruch 3 bis 9, wobei der Grüntee-Extrakt in einer Menge von 30 mg bis 300 mg pro Tag, vorzugsweise von 100 mg bis 200 mg pro Tag Epigallocatechingallat (EGCG), verabreicht wird.

11. Zusammensetzung nach einem der Ansprüche 1 oder 2 oder Zusammensetzung zur Verwendung nach einem der Ansprüche 3 bis 10, wobei es sich bei der Zusammensetzung um eine kosmetische Zusammensetzung, eine Nährstoffzusammensetzung, ein Lebensmittelprodukt, ein Nahrungsergänzungsmittel, ein medizinisches Lebensmittel oder ein Arzneimittel handelt.

12. Zusammensetzung nach einem der Ansprüche 1 oder 2 oder Zusammensetzung zur Verwendung nach einem der Ansprüche 3 bis 11, wobei die Zusammensetzung außerdem einen zweiten Pflanzenextrakt enthält.

13. Zusammensetzung nach Anspruch 1 oder 2 oder Zusammensetzung zur Verwendung nach einem der Ansprüche 3 bis 12, wobei der zweite Pflanzenextrakt ausgewählt ist aus der Gruppe bestehend aus *Aronia melanocarpa, Emblica officinalis, Olea Europa, Citrus bergamia, Vaccinium macrocarpon, Myrciaria dubia,* roter Panax ginseng, *Vaccinium oxycoccos, Vaccinium macrocarpon.*

14. Verfahren zur Herstellung einer Zusammensetzung nach Anspruch 1 oder 2, das die folgenden Schritte umfasst
a. Kultivierung von Zellen der Gattung *Akkermansia;*
b. Pasteurisieren der in Schritt a) erhaltenen kultivierten Zellen;
c. Hinzufügen eines Kälteschutzmittels und eines pH-Puffers
d. Gefriertrocknen der pasteurisierten Zellen aus Schritt c);
e. Mischen des gefriergetrockneten Pulvers mit einem Grüntee-Extrakt; und
f. Wahlweise Herstellung von Kapseln oder Gelkapseln aus der Mischung von Schritt e).

15. Verwendung der Zusammensetzung nach Anspruch 1 oder 2 zur Förderung der Gewichtsabnahme oder zur Verringerung von viszeralem Fett.

## Revendications

1. Composition comprenant de l'*Akkermansia muciniphila* et un extrait de thé vert.

2. Composition selon la revendication 1, dans laquelle *Akkermansia muciniphila* est pasteurisée.

3. Composition selon l'une quelconque des revendications précédentes pour utilisation dans la prévention ou le traitement du dysfonctionnement de la barrière intestinale ou d'un trouble métabolique choisi dans le groupe comprenant l'obésité, le syndrome métabolique, les troubles liés à la carence en insuline ou à la résistance à l'insuline, le diabète sucré, l'intolérance au glucose, le métabolisme lipidique anormal, l'hyperglycémie, la dyslipidémie, les maladies de la barrière intestinale, y compris les maladies inflammatoires de l'intestin, la maladie de Crohn et la colite ulcéreuse, le syndrome du côlon irritable, les troubles de la contractilité intestinale, l'hypercholestérolémie et la dyslipidémie athérogène.

4. Composition à utiliser selon la revendication 3, dans laquelle le trouble métabolique est l'obésité.

5. Composition selon les revendications 1 ou 2 ou composition pour utilisation selon les revendications 3 ou 4, comprenant en outre un ou plusieurs ingrédients choisis dans le groupe constitué par les probiotiques, les bactéries, les levures, les micro-organismes, les prébiotiques ou une combinaison de ceux-ci.

6. Composition selon les revendications 1 ou 2 ou composition pour utilisation selon les revendications 3 à 5, dans laquelle la composition comprend en outre un minéral ou une vitamine ou une combinaison de ceux-ci.

7. Composition selon les revendications 1 ou 2 ou composition pour utilisation selon les revendications 3 à 6, dans laquelle la composition comprend en outre un support pharmaceutiquement acceptable ou un support de qualité alimentaire.

8. Composition selon les revendications 1 ou 2 ou composition pour utilisation selon les revendications 3 à 7, dans laquelle la composition est administrée par voie orale.

9. Composition selon les revendications 1 ou 2 ou composition pour utilisation selon les revendications 3 à 8, dans laquelle l'*Akkermansia muciniphila* pasteurisée est administrée en une quantité allant de 1.10⁴ à 1.10¹² cellules par jour, plus préférentiellement de 1.10⁵ cellules à 1.10¹¹ cellules par jour, et encore plus préférentiellement de 1.10⁶ à 1.10¹⁰ cellules par jour.

10. Composition selon les revendications 1 ou 2 ou composition pour utilisation selon les revendications 3 à 9, dans laquelle l'extrait de thé vert est administré à raison de 30 mg à 300 mg par jour, de préférence de 100 mg à 200 mg par jour de gallate d'épigallocatéchine (EGCG).

11. Composition selon les revendications 1 ou 2 ou composition pour utilisation selon les revendications 3 à 10, dans laquelle la composition est une composition cosmétique, une composition nutritionnelle, un produit alimentaire, un complément alimentaire, un aliment médical ou un médicament.

12. Composition selon les revendications 1 ou 2 ou composition pour utilisation selon les revendications 3 à 11, dans laquelle la composition comprend en outre un deuxième extrait de plante.

13. Composition selon les revendications 1 ou 2 ou composition pour utilisation selon les revendications 3 à 12, dans laquelle le second extrait végétal est choisi dans le groupe constitué par *Aronia melanocarpa, Emblica officinalis, Olea Europa, Citrus bergamia, Vaccinium macrocarpon, Myrciaria dubia,* Panax ginseng rouge, *Vaccinium oxycoccos, Vaccinium macrocarpon.*

14. Procédé de production de la composition selon les revendications 1 ou 2 comprenant les étapes suivantes
a. Culture de cellules du genre *Akkermansia ;*
b. Pasteuriser les cellules cultivées obtenues à l'étape a) ;
c. Ajout d'un cryoprotecteur et d'un tampon pH
d. Lyophiliser les cellules pasteurisées de l'étape c) ;
e. Mélanger la poudre lyophilisée avec un extrait de thé vert ; et
f. Production éventuelle de capsules ou de gélules à partir du mélange de l'étape e).

15. Utilisation de la composition selon les revendications 1 ou 2 pour favoriser la perte de poids ou pour réduire la graisse viscérale.
